# EUROPEAN PATENT APPLICATION

(11) **EP 2 128 889 A1**
(43) Date of publication of application: **02.12.2009**
(21) Application number: 07730388.1
(22) Date of filing: 16.03.2007
(51) Int. Cl.: H01J 61/22

(54) **LIGHT SOURCE WITH REDUCED EMISSION OF SHORT WAVELENGTHS FOR PROTECTION OF THE EYES**

(30) Priority: 07.02.2007 ES 200700328
(71) Applicant: Universidad Complutense de Madrid Rectorado, 28040 Madrid (ES)
(72) Inventor: SANCHEZ RAMOS, Celia, 28040 Madrid (ES)
(74) Representative: Temino Ceniceros, Ignacio
(86) International application number: PCT/ES2007/000147
(87) International publication number: WO 2008/096020

(57) **Abstract**

The subject of the invention is a light source which reduces in a variable percentage the emission of short wavelengths with a view to protecting healthy eyes and/or pseudoaphakic eyes (operated for cataracts) and/or eyes with retinal degeneration of the short wavelengths of the visible spectrum, from 500 to 380nm, whether from incandescent lights, halogen lamps, fluorescent lamps or other sources of emission. This invention avoids the difficulties and risks of existing techniques for protecting healthy eyes, eyes operated for cataracts and improving protection for those in neurodegenerative processes, doing so by a straightforward reduction in the emission of visible spectrum short wavelengths in a source of light. The invention includes a common light source wherein the emission of visible spectrum short wavelengths is reduced by a variable percentage, from 500 to 380nm.

## Description

### Object of the invention

The invention is intended for the ophthalmology sector of the market, within the area of optical applications of a therapeutic and/or prophylactic nature.

The object of this invention is an illumination system that emits a reduced, variable percentage of the short wavelengths of the visible spectrum from 500 to 380 nm. The objective of the invention is to protect healthy eyes and/or pseudoaphakic eyes (eyes that have undergone cataract surgery) and/or eyes with retinal degeneration from the short wavelengths of the visible spectrum, which could provoke neurodegeneration.

### Background of the invention

Visual perception is the result of the response to visible radiation in the wavelength range 380-760 nm. In the environment, solar radiation is the main risk factor for vision. The sun emits UV rays and IR radiation, which are mainly absorbed by the atmosphere. When the solar radiation transmitted through the atmosphere reaches the Earth's surface it consists of UV-B rays (230-300 nm), UV or UV-A rays (300-380 nm), visible light rays (380-760 nm) and IR rays (760-1400 nm). Healthy human eyes freely transmit IR rays and those of most of the visible spectrum to the retina, but the cornea and crystalline lens prevent the most reactive wavelengths of the visible spectrum (UV-B rays and the blue portion of the spectrum) from reaching the retina.

The human crystalline lens changes its transmission properties as it ages by intensifying its yellowish color thus increasing its capacity to filter out UV and blue light rays. Hence, in persons older than 65 years, ultraviolet light (<400 nm) is not transmitted and the transmission of blue light (400-500 nm) is markedly reduced.

The retina is capable of protecting itself from short wavelengths of light in two ways: through its uneven distribution of photoreceptors, such that there are no photoreceptors sensitive to blue light in the macular depression; and through the actions of yellow pigments in this zone, which also exert a protective effect.

These natural protection systems the human eye has against the shorter wavelengths of light--the crystalline lens and structures of the retina--can be seriously affected by certain diseases and/or surgical procedures:
- Cataracts, whose surgical treatment involves the removal of the crystalline lens; and
- Additionally, it is common to find a pathological ageing process that causes degradation of the retinal structures producing age-related macular degeneration (AMD).

We should also consider that both cataracts and AMD can coexist in persons older than 65 years. In this population of elderly subjects, cataract is the main cause of vision loss and AMD is the main cause of blindness. In addition we should expect an increase in both these diseases due, among other factors, to our increased life expectancy. This translates into a great interest in these diseases and their treatment options in the research field and optics industry.

Several epidemiological studies have evaluated the relationship between cataract surgery and AMD. Thus, Klein, et al., The Association Of Cataract And Cataract Surgery With The Long-Term Incidence Of Age-Related Maculopathy, Arch Opthalmol 120:1551-1558 (2002), and Freeman, et al., Is There An Association Between Cataract Surgery And Age-Related Macular Degeneration?, Am J Opthalmolm 135(6): 849-856 (2003), claim there is a higher risk of developing symptoms of AMD in persons who have undergone cataract surgery. However, in earlier investigations by Wang, et al., Cataract And Age-Related Maculopathy: The Blue Mountains Eye Study, Ophthalmic Epidemiol 6: 317-326 (1999) and McCarty, et al., Risks Factors For Age-Related Maculopathy: The Visual Impairment Project, Arch Opthalmol 119:1455-1462 (2001), this hypothesis was rejected, possibly because of the less developed technology used for their diagnostic measurements. Techniques such as optical coherence tomography that allow the accurate, rapid and non-invasive follow up of retinal neurodegeneration processes have only recently been introduced. These techniques are essential for establishing the determining effect of the natural pigments that absorb harmful radiations.

Several techniques have also been developed to protect eyes subjected to cataract surgery from short wavelengths of light:
- There are several types of filter containing a yellow pigment on the market yet there is no optimal procedure and/or device to apply these filters to the human eye as a preventive and/or therapeutic measure to replace and/or improve the eye's natural protection;
   Since the mid-1990s, eyes undergoing cataract extraction have been implanted with intraocular lenses containing a yellow pigment to act as a filter. This option requires surgical intervention with all its risks and difficulties. There is also a large population of subjects who have been implanted with a transparent lens to replace the natural lens during cataract surgery who are therefore devoid of the necessary protection. In these patients, the artificial crystalline lens, lacking a yellow pigment, needs to be complemented with a system to support the yellow pigment, for example, an ophthalmologic lens or contact lens. Whatever the case, retinal damage should be avoided by reducing the proportion of blue and ultraviolet light that reaches the eye. This invention attempts to reduce the incidence of short wavelength light through the reduced emission of the light in the wavelength band under 500 nm.

Several patents related to the state of this technique have been developed although they differ considerably from the object of the present invention:
- Optical and protective element with a flat surface for use in microscopes that illuminates the light ray path (patent JP 2005-349211);
- Lamp for emergency illumination that protects the eye (patent CN 2698995Y);
- Absorption filter for color exposure systems (U.S. Pat. No. 5,121,030) thatthrough the use of dyes--improves visibility in conditions of intense luminosity;
- Special optical filters for certain activities and optical accessories that use these filters (U.S. Pat. No. 6,893,127) to improve the visualization of objects, for example, in sports activities;
- Method for designing color filters that improve or modify the color vision of the human eye, and color filtrating media designed by the method (U.S. Patent Application Publication No. 2004/0075810);
- System and method for applying correction factors related to the environmental conditions (U.S. Patent Application Publication No. 2006/0195278) based on a color detector programmed by software and/or hardware to counteract environmental conditions;
- Protection solution for the treatment of eyes (International Patent Application Publication No. WO 2005/025575) composed mainly of a viscoelastic fluid or rinse containing substances that, at least in part, filter specific frequencies of light radiation;
- Protection and correction device for the human eye that includes a set of filters to protect against electromagnetic radiation and/or corrects visual defects such as myopia or lack of color vision (patent DE 10259261);
- Optical vision system that includes a device for partially reducing the illumination intensity (U.S. Patent Application Publication No. 2002/0113941), as for example a surgical microscope, that includes a spectral filter adapted to reduce, without eliminating, the intensity of light emitted from a light source in a specific region of the object (which could be the human eye);
- System for the detection and control of light intensity for ocular and projecting microscope lamps (U.S. Pat. No. 6,299,310, based on U.S. Pat. No. 4,715,704) that allows work at a high level of illumination of the eye under examination and also avoids damage to the eye being examined;
- Diffusion plate combined with a microscope lamp (patent DE 8808871) that controls the light emitted by the lamp;
- LED system (light emitting diode) for eye examinations (patent IT 1147092) that may incorporate filters;
- Solid photometer--an apparatus for detecting eye and optic nerve defects (patent JP 5130976)--that includes the use of neutral intensity filters;
- Optic lens with selective transmission functions (U.S. patent RE 38,402) in the form of orange spectacles or contact lenses that improve vision and reduce eye damage in luminous environments by substantially reducing ultraviolet and blue light between 400 and 500 nm;
- Polarizing lenses that block blue light and ultraviolet rays (U.S. Pat. No. 5,400,175). The design combines a polarizer, which horizontally blocks polarized light, and a filter, which blocks blue light and ultraviolet radiation;
- Polarized contact lenses (U.S. Pat. No. 6,874,888) with a clear peripheral zone and a polarizing element that covers the pupil area and thus protects eyes against detrimental sunrays or other potentially harmful light sources; and
- Color contact lenses for cataracts (patent JP 11253480) designed to resolve problems with sunglasses. It consists of a pupil like those used in colored contact lenses that reproduces the effects of a sunglass and has a colored patterned iris.

These devices differ from the present invention mainly in their purpose and utility since none has been designed to protect healthy eyes, eyes subjected to cataract surgery or eyes suffering neurodegeneration from short wavelengths of light. Moreover, most of these patents do not refer to a light source, rather they describe other formats: filters, lenses, solutions or others.

### Description of the invention.

The object of this invention is an illumination system that emits a reduced, variable percentage of the short wavelengths of the visible spectrum from 500 to 380 nm. The objective of the invention is to protect healthy eyes and/or pseudoaphakic eyes (eyes that have undergone cataract surgery) and/or eyes with retinal degeneration from the short wavelengths of the visible spectrum, which could provoke neurodegeneration.

For this, the invention consists of a light source that protects healthy, pseudoaphakic and/or eyes suffering neurodegeneration from harmful light rays by reducing to a variable extent the emission of short wavelengths of the visible spectrum from 500 to 380 nm.

### Preferred embodiment of the invention

There are several ways of manufacturing the invention depending on the type of illumination system used. The instructions below are provided as an example, but are in no way restrictive.

Example of manufacturing the invention:
- The elements of an ordinary lamp (contacts, pin and filament) and a translucent polycrystalline ceramic bulb are needed.
- The method followed is that of manufacturing a bulb with a high-sodium vapor content by introducing a mixture of xenon, a mercury amalgam and sodium at a pressure of 200 mm Hg.
- Color reproduction in this type of sodium high-pressure lamp is achieved by mostly emitting yellow-orange light and minimizing the emission of blue and violet light. The chromatic response provided is acceptable for human vision.

In conclusion, the reduced amount of blue/vioiet light emitted by this light source will protect from the effects of short wavelengths of light: the healthy eyes of any subject, the eyes of patients operated on for cataract implanted with a transparent intraocular lens (by supplementing their unprotected artificial lens) and the eyes of persons suffering neurodegeneration (by improving their natural protection). This system avoids the problems related to the options available on the market (filters with no support system, intraocular lenses) yet still protects against the harmful effects of blue light.

## Claims

1. Illumination source to protect eyes comprising a light source and an element that reduces, in a variable percentage, the emission of short wavelengths of the visible spectrum.

2. Illumination source to protect eyes according to claim 1, wherein the light source is one of incandescent, halogen, fluorescent light source or other source with any emission method.

3. Illumination source to protect eyes according to claim 1, wherein the emission of short wavelengths of the visible spectrum is reduced by appropriately combining the products that project light.

4. Illumination source to protect eyes according to claim 1, wherein the reduction of the emission of short wavelengths of the visible spectrum is variable from 0% to 100%.

5. Illumination source to protect eyes according to claim 1, wherein the reduced wavelengths of the visible spectrum are between about 500 nm to about 380 nm.

6. Use of the illumination source of claims 1 to 5 for protecting healthy eyes.

7. Use of the illumination source of claims 1 to 5 for protecting pseudoaphakic eyes.

8. Use of the illumination source of claims 1 to 5 for protecting eyes that are suffering neurodegeneration.
